(19) Europäisches Patentamt
European Patent Office
Office européen des brevets

(11) Numéro de publication: 0 032 856
B1

(12) FASCICULE DE BREVET EUROPEEN

(45) Date de publication du fascicule du brevet: 09.02.83

(51) Int Cl.³: C 07 D 487/04, A 61 K 31/50 // (C07D487/04, 237/00, 235/00)

(21) Numéro de dépôt: 81400036.0

(22) Date de dépôt: 13.01.81

(54) **Nouveaux dérivés de dihydro-2,3-imidazo(1,2-b)pyridazines, leur procédé de préparation et leur application en thérapeutique.**

(30) Priorité: **16.01.80 FR 8000926**

(43) Date de publication de la demande: **29.07.81 Bulletin 81/30**

(45) Mention de la délivrance du brevet: **09.02.83 Bulletin 83/6**

(84) Etats contractants désignés: **AT BE CH DE FR GB IT LI LU NL SE**

(56) Documents cités: **DE-A-2 741 763**
**FR-A-2 380 281**

(73) Titulaire: **SYNTHELABO, 58 rue de la Glacière, F-75013 Paris (FR)**

(72) Inventeur: **Fabiani, Paul, 9, avenue Céline, F-92200 Neuilly (FR)**
Inventeur: **Rose, Francis, 6, rue des Haudriettes, F-75003 Paris (FR)**
Inventeur: **Vartanian, Abkar, 104, rue des Lilas, F-45200 Amilly (FR)**
Inventeur: **Warolin, Christian, 55, Bd Beauséjour, F-75016 Paris (FR)**

(74) Mandataire: **Thouret-Lemaitre, Elisabeth, Service Brevets - SYNTHELABO 58, rue de la Glacière, F-75621 Paris Cedex 13 (FR)**

EP 0 032 856 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés de dihydro-2,3-imidazo(1,2-b)pyridazines, leur procédé de préparation et leur application en thérapeutique

La présente invention concerne de nouveaux dérivés de dihydro-2,3-imidazo[1,2-b]pyridazines, leur procédé de préparation et leur application en thérapeutique.

Les composés selon l'invention répondent à la formule générale (I)

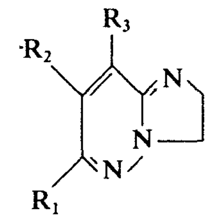

(I)

dans laquelle

$R_1$ représente un radical phényle, halogénophényle, trifluorométhylphényle, $(C_{1-4})$-alkylphényle, $(C_{1-4})$-alcoxyphényle, hydroxyphényle, nitrophényle, aminophényle, $(C_{1-4})$-alkylaminophényle, di-$(C_{1-4})$-alkylaminophényle, acétamidophényle, thiényle, pyridyle, furyle ou nitrofuryle,

$R_2$ représente un atome d'hydrogène ou un radical $(C_{1-4})$-alkyle,

$R_3$ représente un atome d'hydrogène ou un radical $(C_{1-4})$-alkyle, phényle, halogénophényle, trifluorométhylphényle, $(C_{1-4})$-alkylphényle, $(C_{1-4})$-alcoxyphényle, hydroxyphényle, nitrophényle, aminophényle, $(C_{1-4})$-alkylaminophényle, di-$(C_{1-4})$-alkylaminophényle ou acétamidophényle.

L'invention concerne également les sels d'addition des composés (I) aux acides pharmaceutiquement acceptables.

Les composés préférés de formule générale (I) sont ceux pour lesquels $R_1$ est un radical aminophényle, méthylaminophényle, éthylaminophényle, diméthylaminophényle, méthoxyphényle, chlorophényle ou un radical furyle,

$R_2$ est un atome d'hydrogène ou un radical méthyle et $R_3$ est un atome d'hydrogène, un radical méthyle, phényle ou chlorophényle.

Les composés (I) plus particulièrement préférés sont ceux pour lesquels $R_2$ et $R_3$ représentent chacun un atome d'hydrogène et $R_1$ représente un radical aminophényle, méthylaminophényle, éthylaminophényle ou furyle.

Les composés (I) sont préparés par cyclisation d'un composé de formule générale (II)

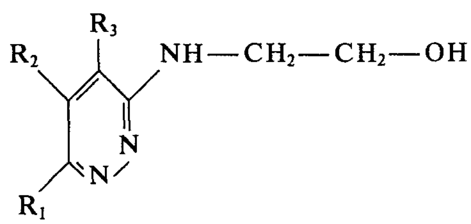

(II)

dans laquelle

$R_1$, $R_2$ et $R_3$ ont les significations mentionnées plus haut.

La cyclisation conduisant aux composés (I) est effectuée soit par traitement du composé (II) avec de l'acide polyphosphorique à une température de 100 à 250°C, soit par traitement du composé (II) avec du chlorure de thionyle dans un solvan te, tel que le chloroforme, à la température de reflux suivi, après évaporation, d'un chauffage à empérature de reflux dans de l'éthanol en présence de carbonate de potassium ou de bicarbonate de sodium.

Les composés (I) pour lesquels $R_1$ représente un radical dialkylaminophényle peuvent également être préparés à partir du composé (I) correspondant pour lequel $R_1$ est un radical aminophényle, selon une méthode classique d'alkylation.

Les composés (I) pour lesquels $R_1$ représente un radical acétamidophényle peuvent également être préparés à partir d'un composé (I) pour lequel $R_1$ est un radical aminophényle selon une méthode classique d'acétylation.

Les exemples suivants illustrent l'invention.

Les analyses et les spectres IR et RMN ont confirmé la structure des composés.

## Exemple 1

### (Amino-4 phenyl)-6 dihydro-2,3 imidazo[1,2-b]pyridazine et son chlorhydrate

On porte à 150°C un mélange de 16,1 g (0,07 mole) d'(amino-4 phényl)-6-(hydroxy-2 éthylamino)-3-pyridazine et de 185 g d'acide polyphosphorique. On maintient la température entre 150

et 160° C pendant 30 minutes. On refroidit le mélange vers 100° C et on le verse dans 400 ml d'eau froide. La solution obtenue refroidie dans un bain de glace et est alcalinisée avec 400 ml de soude (environ 10 N). Le produit qui a précipité est essoré, lavé avec de l'eau et séché sous vide. On obtient la base après purifications.
F = 259° C.

On dissout 6,5 g de ce produit (0,03 mole) dans 350 ml de méthanol au reflux. On filtre la solution chaude, puis on ajoute un équivalent d'acide chlorhydrique éthanolique. Le chlorhydrate précipite. Après refroidissement, on essore le produit, on le lave avec du méthanol et on le sèche.
F = 302 – 303° C. (décomposition).

Exemple 2

(Furyl-2)-6 dihydro-2,3 imidazo[1,2-b]pyridazine et son chlorhydrate

Dans un ballon de 500 ml et sous agitation magnétique, on place 200 ml de chloroforme puis 5,5 g (0,0268 mole) de (furyl-2)-6 (hydroxy-2 éthyl) amino-3 pyridazine. On ajoute lentement, en 10 minutes, 5 ml de chlorure de thionyle puis on porte au reflux pendant 1h 30. Il se produit la cristallisation d'un produit crème. On concentre à sec au rotavapor, puis on ajoute 150 ml d'éthanol absolu. Toujours, sous agitation magnétique, la solution éthanolique est portée une heure à reflux avec 10 g de carbonate de potassium. On essore et on concentre la solution éthanolique à sec. On obtient la base.
F = 223° C.

La base est reprise par un mélange de 50 ml d'éthanol et de 20 ml de méthanol, et on ajoute 5 ml d'acide chlorhydrique éthanolique 5N. On traite au charbon à chaud, on filtre à chaud, puis on laisse refroidir à la température ambiante, puis au réfrigérateur une nuit. On essore, on rince à l'alcool et on sèche à l'étuve à 70° C sous vide.
F = 265 – 268° C.

Exemple 3

(Diméthylamino-4 phényl)-6 dihydro-2,3 imidazo[1,2-b]pyridazine et son chlorhydrate

Dans un ballon de 100 ml muni d'un agitateur magnétique, on introduit 1,52 g (0,0072 mole) d'(amino-4 phényl)-6 dihydro-2,3 imidazo[1,2-]pyridazine imidazo[1,2-bpyridazine préparée selon l'exemple 1. On ajoute 6,2 ml de formol à 37%. Lorsque la dissolution est complète, on ajoute 1,37 g (0,0232 mole) de cyanoborohydrure de sodium, puis, goutte à goutte en 7 minutes, 0,77 ml d'acide acétique. La température du milieu s'élève. On agite pendant deux heures. On ajoute ensuite 0,77 ml d'acide acétique et on agite encore une heure. On évapore à sec au rotavapor, on reprend par 50 ml de chloroforme et on lave avec 50 ml de soude 1N puis avec deux fois 50 ml d'eau. Le produit est extrait avec 50 ml d'acide chlorhydrique normal et on lave avec 50 ml de chloroforme. La phase aqueuse est alcalinisée avec de la soude 1N. Le précipité est essoré, lavé à l'eau et séché. On dissout 1,6 g de la base obtenue dans 50 ml d'éthanol tiède, on ajoute un équivalent d'acide chlorhydrique dans l'éthanol puis 75 ml d'éther. Le précipité est essoré et séché.
F = 259 – 261° C.

Dans le tableau (I) suivant sont représentés les composés de l'invention préparés à titre d'exemples.

Tableau I

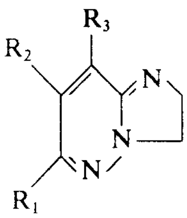

B = base
HCl = chlorhydrate

| N° | $R_1$ | $R_2$ | $R_3$ | base ou sel | F (°C) |
|---|---|---|---|---|---|
| 1 | 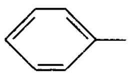 | H | H | HCl | 265 – 6 |
| 2 | 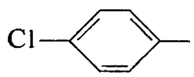 | H | H | HCl | 255 – 7 |
| 3 (Ex 1) | 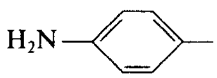 | H | H | HCl | 302 – 3 (décomp.) |
| 4 | 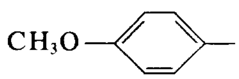 | H | H | HCl | 245 – 7 |
| 5 | 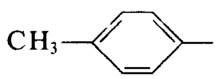 | H | H | HCl | 255 – 60 |
| 6 | 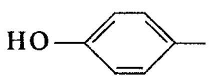 | H | H | B | 270 – 2 |
| 7 | 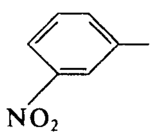 | H | H | HCl | 275 – 7 |
| 8 | 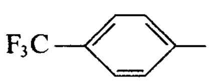 | H | H | HCl | 250 – 2 |
| 9 | 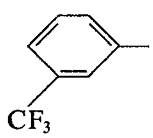 | H | H | HCl | 256 |
| 10 | 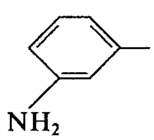 | H | H | HCl | 295 – 7 (décomp.) |
| 11 | 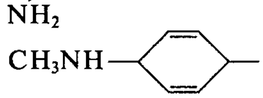 | H | H | HCl | 292 – 4 |
| 12 (EX 3) | 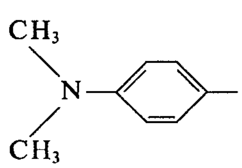 | H | H | HCl | 259 – 61 |
| 13 | 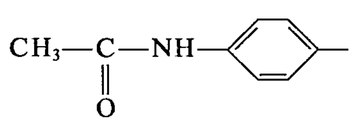 | H | H | HCl | 296 – 7 |

Fortsetzung

| N° | $R_1$ | $R_2$ | $R_3$ | base ou sel | F (°C) |
|---|---|---|---|---|---|
| 14 | 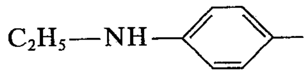 | H | H | HCl | 243–4 |
| 15 | 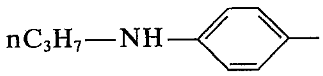 | H | H | HCl | 230–2 |
| 16 | 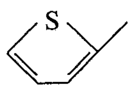 | H | H | HCl | 268–70 (décomp.) |
| 17 | 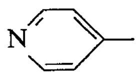 | H | H | HCl | 285–90 (décomp.) |
| 18 (EX 2) | 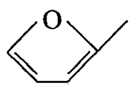 | H | H | HCl | 265–8 |
| 19 | 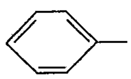 | H | 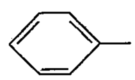 | HCl<br>B | 286–8<br>145 |
| 20 | 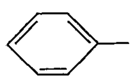 | H | 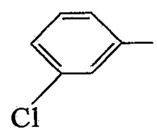 | HCl | 242–4 |
| 21 | 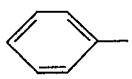 | H | 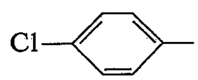 | B | 144 |
| 22 | 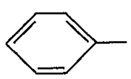 | H | 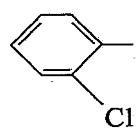 | B | 143 |
| 23 | 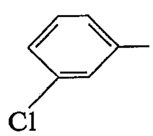 | H | 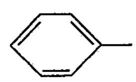 | HCl | 223–7 |
| 24 | 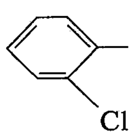 | H | 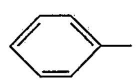 | HCl | 261–3 |
| 25 | 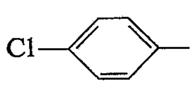 | H | 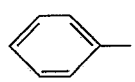 | HCl | 253–5 |
| 26 | 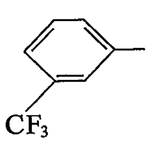 | H | 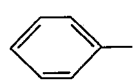 | HCl | 254–5 |
| 27 | 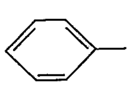 | H | $CH_3$ | HCl | 250 |
| 28 | 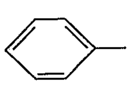 | H | $C_2H_5$ | HCl | 233–5 |

Fortsetzung

| N° | $R_1$ | $R_2$ | $R_3$ | base ou sel | F (°C) |
|---|---|---|---|---|---|
| 29 | 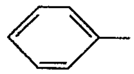 | $CH_3$ | H | HCl | 235 |
| 30 | 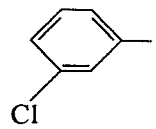 | H | $CH_3$ | HCl | 240 – 1 |
| 31 | 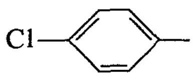 | H | $CH_3$ | HCl | 310 – 2 |
| 32 | 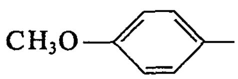 | H | $CH_3$ | HCl | 250 |
| 33 | 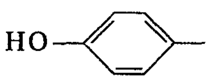 | H | $CH_3$ | HCl | 328 – 30 |
| 34 | 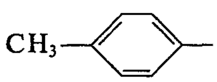 | H | $CH_3$ | HCl | 348 |
| 35 | 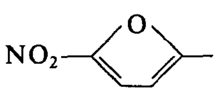 | H | H | HCl | 284 – 286 (déc) |

Les composés de l'invention ont été soumis à des essais pharmacologiques qui ont mis en évidence leurs propriétés antidépressives.

La toxicité des composés a été déterminée sur la souris par voie intraveineuse. La DL 50 va d'environ 50 à 60 mg/kg.

L'activité antidépressive a été déterminée selon le test de l'antagonisme vis à vis de la ptose réserpinique.

Les souris (mâles, CD1 Charles River, France, 18 – 22 g) reçoivent simultanément les produits à étudier ou le solvant (voie i.p.), et la réserpine (4 mg/kg, voie s.c.).

Soixante minutes plus tard, le degré de ptose palpébrale est estimé au moyen d'une échelle de cotation (0 à 4), pour chaque souri.

A chaque dose, la moyenne de cotation et le pourcentage de variation par rapport au lot contrôle, sont calculés.

Pour chaque produit, la DA 50, ou dose qui diminue de 50% le score moyen de ptose par rapport aux contrôles, est déterminée graphiquement.

La DA 50 varie de 0,05 à 1 mg/kg par voie i.p.

Les résultats des tests pharmacologiques montrent que les composés de l'invention peuvent être utiles pour le traitement de la dépression, en thérapeutique humaine.

Les composés de l'invention peuvent être présentés sous toute forme appropriée pour l'administration par voie orale, parentérale, endorectale, par exemple sous forme de comprimés, de dragées, de gélules, de solutions buvables ou injectables, etc. . . avec tout excipient approprié.

La posologie orale quotidienne peut aller de 50 à 500 mg.

**Revendications pour les Etats contractants: BE, CH, DE, FR, GB, IT, LI, LU, NL, SE**

1. Composés répondant à la formule générale (I)

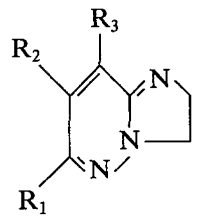

dans laquelle
$R_1$ représente un radical phényle, halogénophényle, trifluorométhylphényle, $(C_{1-4})$-alkylphényle, $(C_{1-4})$-alcoxyphényle, hydroxyphényle, nitrophényle, aminophényle, $(C_{1-4})$-alkylaminophényle, di$(C_{1-4})$-alkylaminophényle, acétamidophényle, thiényle, pyridyle, furyle ou nitrofuryle,
$R_2$ représente un atome d'hydrogène ou un radical $(C_{1-4})$-alkyle,
$R_3$ représente un atome d'hydrogène ou un radical $(C_{1-4})$-alkyle, phényle, halogénophényle, trifluorométhylphényle, $(C_{1-4})$-alkylphényle, $(C_{1-4})$-alcoxyphényle, hydroxyphényle, nitrophényle, aminophényle, $(C_{1-4})$-alkylaminophényle, di-$(C_{1-4})$-alkylaminophényle ou acétamidophényle,
ainsi que leurs sels d'addition aux acides pharmaceutiquement acceptables.

2. Composés selon la revendication 1, pour lesquels $R_1$ représente un radical phényle, chlorophényle, trifluorométhylphényle, méthylphényle, méthoxyphényle, hydroxyphényle, nitrophényle, aminophényle, méthylaminophényle, éthylaminophényle, propylaminophényle, diméthylaminophényle, acétamidophényle, thiényle, pyridyle ou furyle,
$R_2$ représente un atome d'hydrogène ou un radical $(C_{1-4})$-alkyle et
$R_3$ représente un atome d'hydrogène ou un radical $(C_{1-4})$-alkyle, phényle ou halogénophényle.

3. Composés selon la revendication 2, pour lesquels $R_1$ est un radical aminophényle, méthylaminophényle, éthylaminophényle, diméthylaminophényle, méthoxyphényle, chlorophényle ou furyle,
$R_2$ est un atome d'hydrogène ou un radical méthyle et
$R_3$ est un atome d'hydrogène ou un radical méthyle, phényle ou chlorophényle.

4. Composés selon la revendication 3, pour lesquels $R_1$ est un radical aminophényle, méthylaminophényle, éthylaminophényle ou furyle, et $R_2$ et $R_3$ sont chacun un atome d'hydrogène.

5. L'(amino-4 phényl)-6 dihydro-2,3 imidazo[1,2-b]pyridazine et ses sels pharmaceutiquement acceptables.

6. La (méthylamino-4 phényl)-6 dihydro-2,3 imidazo[1,2-b]pyridazine et ses sels.

7. La (furyl-2)-6 dihydro-2,3 imidazo[1,2-b]pyridazine et ses sels.

8. La (méthoxy-4 phényl)-6 dihydro-2,3 imidazo[1,2-b]pyridazine et ses sels pharmaceutiquement acceptables.

9. L'(éthylamino-4 phényl)-6 dihydro-2,3 imidazo[1,2-b]pyridazine et ses sels pharmaceutiquement acceptables.

10. Procédé de préparation des composés de formule générale (I), procédé caractérisé en ce que l'on cyclise un composé de formule générale II

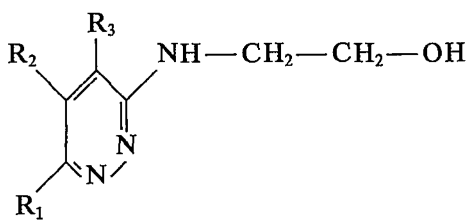

(II)

dans laquelle
$R_1$, $R_2$ et $R_3$ ont les significations mentionnées dans la revendication 1, soit par traitement avec de l'acide polyphosphorique à chaud, soit par traitement avec du chlorure de thionyle dans un solvant inerte, tel que le chloroforme, à la température de reflux suivi, après évaporation, d'un chauffage à la température de reflux dans de l'éthanol, en présence d'un carbonate ou d'un bicarbonate alcalin.

11. Composition pharmaceutique caractérisée en ce qu'elle contient un composé tel que spécifié dans l'une quelconque des revendications 1 à 9.

12. Médicament caractérisé en ce qu'il contient un composé spécifie dans l'une quelconque des revendications 1 à 9.

**Revendication pour l'Etat contractant: AT**

Procédé de préparation des composés répondant à la formule générale (I),

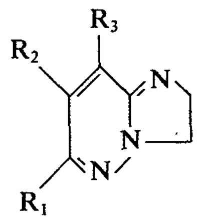

(I)

dans laquelle
$R_1$ représente un radical phényle, halogénophényle, trifluorométhylphényle, $(C_{1-4})$-alkylphényle, $(C_{1-4})$-alcoxyphényle, hydroxyphényle, nitrophényle, aminophényle, $(C_{1-4})$-alkylaminophényle, di-$(C_{1-4})$-alkylaminophényle, acétamidophényle, thiényle, pyridyle, furyle ou nitrofuryle,
$R_2$ représente un atome d'hydrogène ou un radical $(C_{1-4})$-alkyle,
$R_3$ représente un atome d'hydrogène ou un radical $(C_{1-4})$-alkyle, phényle, halogénophényle, trifluorométhylphényle, $(C_{1-4})$-alkylphényle, $(C_{1-4})$-alcoxyphényle, hydroxyphényle, nitrophényle, aminophényle, $(C_{1-4})$-alkylaminophényle, di-$(C_{1-4})$-alkylaminophényle ou acétamidophényle,
ainsi que de leurs sels d'addition aux acides pharmaceutiquement acceptables,
procédé caractérisé en ce que l'on cyclise un composé de formule générale (II)

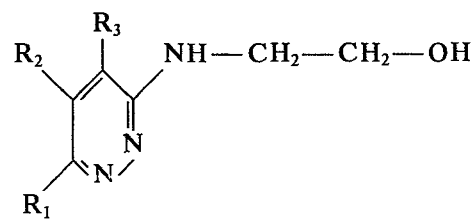

(II)

dans laquelle
$R_1$, $R_2$ et $R_3$ ont les significations mentionnées ci-dessus,
soit par traitement avec de l'acide polyphosphorique à chaud, soit par traitement avec du chlorure de thionyle dans un solvant inerte, tel que le chloroforme, à la température de reflux suivi, après évaporation, d'un chauffage à la température de reflux dans de l'éthanol, en présence d'un carbonate ou d'un bicarbonate alcalin.

**Patentansprüche für die Vertragsstaaten: BE, CH, DE, FR, GB, IT, LI, LU, NL, SE**

1. Verbindungen der allgemeinen Formel (I)

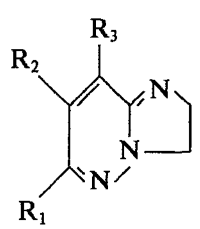

in welcher
$R_1$ einen Phenyl-, Halogenphenyl-, Trifluormethylphenyl-, $(C_{1-4})$-Alkylphenyl-, $(C_{1-4})$-Alkoxyphenyl-, Hydroxyphenyl-, Nitrophenyl-, Aminophenyl-, $(C_{1-4})$-Alkylaminophenyl-, Di-$(C_{1-4})$-alkylaminophenyl-, Acetamidophenyl-, Thienyl-, Pyridyl-, Furyl- oder Nitrofurylrest,
$R_2$ ein Wasserstoffatom oder einen $(C_{1-4})$-Alkylrest und
$R_3$ ein Wasserstoffatom oder einen $(C_{1-4})$-Alkyl-, Phenyl-, Halogenphenyl-, Trifluormethylphenyl-, $(C_{1-4})$-Alkylphenyl-, $(C_{1-4})$-Alkoxyphenyl-, Hydroxyphenyl-, Nitrophenyl-, Aminophenyl-, $(C_{1-4})$-Alkylaminophenyl-, Di-$(C_{1-4})$-alkylaminophenyl- oder Acetamidophenylrest bedeutet,
sowie deren Additionssalze mit pharmazeutisch anwendbaren Säuren.

2. Verbindungen nach Anspruch 1, bei welchen $R_1$ einen Phenyl-, Chlorphenyl-, Trifluormethylphenyl-, Methylphenyl-, Methoxyphenyl-, Hydroxyphenyl-, Nitrophenyl-, Aminophenyl-, Methylaminophenyl-, Ethylaminophenyl-, Propylaminophenyl-, Dimethylaminophenyl-, Acetamidophenyl-, Thienyl-, Pyridyl- oder Furylrest
$R_2$ ein Wasserstoffatom oder einen $(C_{1-4})$-Alkylrest und

$R_3$ ein Wasserstoffatom oder einen $(C_{1-4})$-Alkyl-, Phenyl- oder Halogenphenylrest bedeutet.

3. Verbindungen nach Anspruch 2, bei welchen $R_1$ einen Aminophenyl-, Methylaminophenyl-, Ethylaminophenyl-, Dimethylaminophenyl-, Methoxyphenyl-, Chlorphenyl- oder Furylrest,
$R_2$ ein Wasserstoffatom oder einen Methylrest und
$R_3$ ein Wasserstoffatom oder einen Methyl-, Phenyl- oder Chlorphenylrest bedeutet.

4. Verbindungen nach Anspruch 3, bei welchen $R_1$ einen Aminophenyl-, Methylaminophenyl-, Ethylaminophenyl- oder Furylrest und $R_2$ und $R_3$ jeweils ein Wasserstoffatom bedeutet.

5. 6-(4-Aminophenyl)-2,3-dihydroimidazo[1,2-b]pyridazin und seine pharmazeutisch anwendbaren Salze.

6. 6-(4-Methylaminophenyl)-2,3-dihydroimidazo[1,2-b]pyridazin und seine Salze.

7. 6-(2-Furyl)-2,3-dihydroimidazo[1,2-b]pyridazin und seine Salze.

8. 6-(4-Methoxyphenyl)-2,3-dihydroimidazo[1,2-b]pyridazin und seine pharmazeutisch anwendbaren Salze.

9. 6-(4-Ethylaminophenyl)-2,3-dihydroimidazo[1,2-b]pyridazin und seine pharmazeutisch anwendbaren Salze.

10. Verfahren zur Herstellung von Verbindungen der allgemeinen Formel (I), dadurch gekennzeichnet, daß man eine Verbindung der allgemeinen Formel II

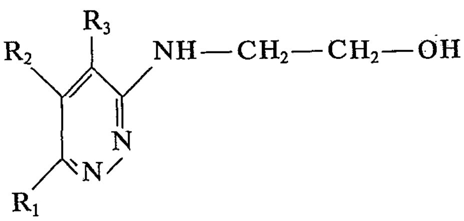

(II)

in welcher $R_1$, $R_2$ und $R_3$ die in Anspruch 1 angegebenen Bedeutungen haben, entweder durch Behandlung mit Polyphosphorsäure in der Hitze oder durch Behandlung mit Thionylchlorid in einem inerten Lösungsmittel, wie Chloroform, bei Rückflußtemperatur zyklisiert, worauf man nach dem Eindampfen anschließend in Ethanol in Gegenwart eines Alkalicarbonats oder -bicarbonats auf Rückflußtemperatur erhitzt.

11. Pharmazeutische Zusammensetzung, dadurch gekennzeichnet, daß sie eine in einem der Ansprüche 1 bis 9 angegebene Verbindung enthält.

12. Arzneimittel, dadurch gekennzeichnet, daß es eine in einem der Ansprüche 1 bis 9 angegebene Verbindung enthält.

**Patentanspruch für den Vertragsstaat: AT**

Verfahren zur Herstellung von Verbindungen der allgemeinen Formel (I)

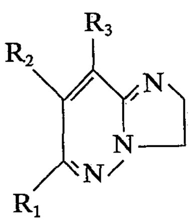

(I)

in welcher $R_1$ einen Phenyl-, Halogenphenyl-, Trifluormethylphenyl-, $(C_{1-4})$-Alkylphenyl-, $(C_{1-4})$-Alkoxyphenyl-, Hydroxyphenyl-, Nitrophenyl-, Aminophenyl-, $(C_{1-4})$-Alkylaminophenyl-, Di-$(C_{1-4})$-Alkylaminophenyl-, Acetamidophenyl-, Thienyl-, Pyridyl-, Furyl- oder Nitrofurylrest,
$R_2$ ein Wasserstoffatom oder einen $(C_{1-4})$-Alkylrest und
$R_3$ ein Wasserstoffatom oder einen $(C_{1-4})$-Alkyl-, Phenyl-, Halogenphenyl-, Trifluormethylphenyl-, $(C_{1-4})$-Alkylphenyl-, $(C_{1-4})$-Alkoxyphenyl-, Hydroxyphenyl-, Nitrophenyl-, Aminophenyl-, $(C_{1-4})$-Alkylaminophenyl-, Di$(C_{1-4})$-Alkylaminophenyl- oder Acetamidophenylrest bedeutet,
sowie deren Additionssalzen mit pharmazeutisch anwendbaren Säuren, dadurch gekennzeichnet, daß

man eine Verbindung der allgemeinen Formel (II)

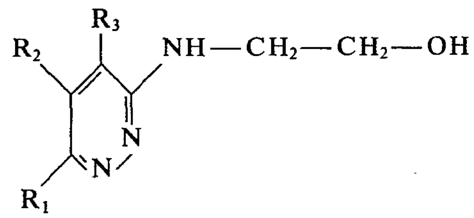

(II)

in welcher $R_1$, $R_2$ und $R_3$ die oben angegebenen Bedeutungen haben, entweder durch Behandlung mit Polyphosphorsäure in der Hitze oder durch Behandlung mit Thionylchlorid in einem inerten Lösungsmittel, wie Chloroform, bei Rückflußtemperatur zyklisiert, worauf man nach dem Eindampfen anschließend in Ethanol in Gegenwart eines Alkalicarbonats oder -bicarbonats auf Rückflußtemperatur erhitzt.

**Claims for the contracting states: BE, CH, DE, FR, GB, IT, LI, LU, NL, SE**

1. Compounds of general formula (I)

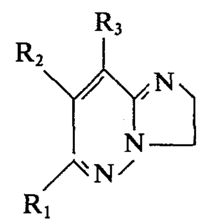

(I)

wherein
$R_1$ is a phenyl, halophenyl, trifluoromethylphenyl, $(C_{1-4})$alkylphenyl, $(C_{1-4})$alkoxyphenyl, hydroxyphenyl, nitrophenyl, aminophenyl, $(C_{1-4})$alkylaminophenyl, di$(C_{1-4})$alkylaminophenyl, acetamidophenyl, thienyl, pyridyl, furyl or nitrofuryl radical,
$R_2$ is a hydrogen atom or a $(C_{1-4})$alkyl radical,
$R_3$ is a hydrogen atom or a $(C_{1-4})$alkyl, phenyl, halophenyl, trifluoromethylphenyl, $(C_{1-4})$alkylphenyl, $(C_{1-4})$alkoxyphenyl, hydroxyphenyl, nitrophenyl, aminophenyl, $(C_{1-4})$alkylaminophenyl, di$(C_{1-4})$alkylaminophenyl or acetamidophenyl radical,
and the addition salts thereof with pharmaceutically acceptable acids.

2. Compounds according to claim 1, wherein
$R_1$ is a phenyl, chlorophenyl, trifluoromethylphenyl, methylphenyl, methoxyphenyl, hydroxyphenyl, nitrophenyl, aminophenyl, methylaminophenyl, ethylaminophenyl, propylaminophenyl, dimethylaminophenyl, acetamidophenyl, thienyl, pyridyl or furyl radic l,
$R_2$ is a hydrogen atom or a $(C_{1-4})$alkyl radical, and
$R_3$ is a hydrogen atom or a $(C_{1-4})$alkyl, phenyl or halophenyl radical.

3. Compounds according to claim 2, wherein
$R_1$ is an aminophenyl, methylaminophenyl, ethylaminophenyl, dimethylaminophenyl methoxyphenyl, chlorophenyl or furyl radical,
$R_2$ is a hydrogen atom or a methyl radical, and
$R_3$ is a hydrogen atom or a methyl, phenyl or chlorophenyl radical.

4. Compounds according to claim 3, wherein
$R_1$ is an aminophenyl, methylaminophenyl, ethylaminophenyl or furyl radical, and
each of $R_2$ and $R_3$ is a hydrogen atom.

5. The 6-(4-aminophenyl)-2,3-dihydro-imidazo[1,2-b]pyridazine and its pharmaceutically acceptable salts.

6. The 6-(4-methylaminophenyl)-2,3-dihydro-imidazo[1,2-b]pyridazine and its salts.

7. The 6-(furyl-2)-2,3-dihydro-imidazo[1,2-b]pyridazine and its salts.

8. The 6-(4-methoxyphenyl)-2,3-dihydro-imidazo[1,2-b]pyridazine and its pharmaceutically acceptable salts.

9. The 6-(4-ethylaminophenyl)-2,3-dihydro-imidazo[1,2-b]pyridazine and its pharmaceutically acceptable salts.

10. A process for the preparation of the compounds of general formula (I), characterized in that a compound of general formula (II)

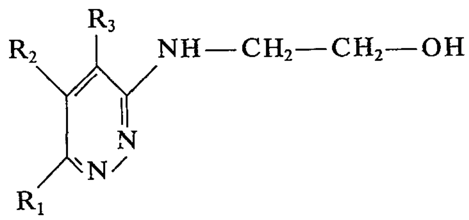

(II)

wherein $R_1$, $R_2$ and $R_3$ are as defined in claim 1, is cyclized either by treatment with polyphosphoric acid at elevated temperature, or by treatment with thionyl chloride in an inert solvent, such as chloroform, at the reflux temperature followed, after evaporation, by heating in ethanol at the reflux temperature, in the presence of an alcali metal carbonate or bicarbonate.

11. Pharmaceutical composition characterized in that it contains a compound as specified in anyone of claims 1 to 9.

12. A drug characterized in that it contains a compound as specified in anyone of claims 1 to 9.

**Claim for the contracting state: AT**

A process for the preparation of the compounds of general formula (I)

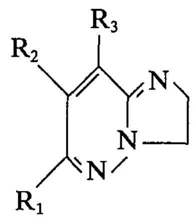

(I)

wherein
$R_1$ is a phenyl, halophenyl, trifluoromethylphenyl, $(C_{1-4})$alkylphenyl, $(C_{1-4})$alkoxyphenyl, hydroxyphenyl, nitrophenyl, aminophenyl, $(C_{1-4})$alkylaminophenyl, di$(C_{1-4})$alkylaminophenyl, acetamidophenyl, thienyl, pyridyl, furyl or nitrofuryl radical,
$R_2$ is a hydrogen atom or a $(C_{1-4})$alkyl radical,
$R_3$ is a hydrogen atom or a $(C_{1-4})$alkyl, phenyl, halophenyl, trifluoromethylphenyl, $(C_{1-4})$alkylphenyl, $(C_{1-4})$alkoxyphenyl, hydroxyphenyl, nitrophenyl, aminophenyl, $(C_{1-4})$alkylaminophenyl, di$(C_{1-4}$alkylaminophenyl or acetamidophenyl radical,
and the addition salts thereof with pharmaceutically acceptable acids,
characterized in that a compound of general formula (II)

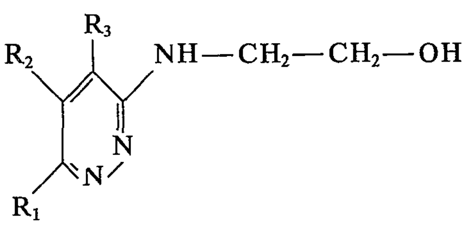

(II)

wherein $R_1$, $R_2$ and$R_3$ are as defined above, is cyclized either by treatment with polyphosphoric acid at elevated temperature, or by treatment with thionyl chloride in an inert solvent, such as chloroform, at the reflux temperature followed, after evaporation, by heating in ethanol at the reflux temperature, in the presence of an alcali metal carbonate or bicarbonate.